# EUROPEAN PATENT APPLICATION

(11) **EP 4 725 499 A1**
(43) Date of publication of application: **15.04.2026**
(21) Application number: 24745882.1
(22) Date of filing: 11.05.2024
(51) Int. Cl.: A61K 39/00, A61P 25/28, C07K 14/47, A61K 38/16

(54) **MULTITARGET CHIMERIC PROTEIN FOR IMMUNOTHERAPY OF ALZHEIMER'S DISEASE**

(30) Priority: 12.05.2023 CU 20230023
(71) Applicant: Centro De Neurociencias De Cuba, 11300 Playa La Habana (CU); Centro de Ingeniería Genética y Biotecnología, 11300 Playa La Habana (CU)
(72) Inventor: LEÓN ARCIA, Karen, La Habana (CU); QUINTERO ALVAREZ, Heidi, La Habana (CU); MORERA DÍAZ, Yanelys, La Habana (CU); PÉREZ LEAL, Gabriela, La Habana (CU); PONCE VALDESPINO, Roberto Yoanky, La Habana (CU); LÓPEZ ARMENTEROS, Mailen, La Habana (CU); BEQUET ROMERO, Mónica, La Habana (CU); ZAMORA LOYARTE, Diana Iris, La Habana (CU); VÁZQUEZ MOJENA, Yaimee, La Habana (CU)
(74) Representative: V.O.
(86) International application number: PCT/CU2024/050004
(87) International publication number: WO 2024/235367

(57) **Abstract**

The present invention is related to the biomedical and biopharmaceutical sectors. Specifically, it refers to a chimeric antigen comprising the combination of the amino and carboxyl terminal regions of the amyloid beta peptide (Aβ), the amino and carboxyl terminal regions of the tau protein and a T cell epitope. The pharmaceutical composition comprising this chimeric antigen and at least one pharmaceutically acceptable vaccine adjuvant increases the efficacy of immunotherapy for the prevention and treatment of Alzheimer's Disease (AD). The chimeric antigen exerts its action by stimulating a multitarget humoral response with high titers of anti-Aβ and anti-tau antibodies simultaneously. This favors the combined elimination of toxic species of both Aβ and tau from the brain, which prevents or significantly improves the clinical symptoms and neuropathology of AD.

## Description

The present invention is related to the biomedical and biopharmaceutical sectors. Specifically, it refers to a chimeric antigen comprising the combination of the B cell epitopes and the carboxyl terminal region of the amyloid beta peptide (Aβ), the amino and carboxyl terminal regions of the tau protein and a T cell epitope. The pharmaceutical composition comprising this chimeric antigen and at least one pharmaceutically acceptable vaccine adjuvant increases the efficacy of immunotherapy for the prevention and treatment of Alzheimer's disease (AD).

AD is the most common form of dementia, the leading cause of disability in adults over 65 years (Tahami Monfared et al., Neurology and therapy, 2022) and the sixth cause of death in the world (Rayathala et al., Journal of Innovations in Applied Pharmaceutical Science, 2022 :7: 28-31). It is a multifactorial neurodegenerative disease for which there is no effective treatment yet. The hallmarks of AD are the accumulation of amyloid beta (Aβ) plaques and neurofibrillary tangles in the brain. Although the exact molecular mechanisms that trigger this pathology are not known, most experts agree that it begins with the accumulation of Aβ peptides due to an imbalance between their production and clearance from the brain (Chen et al., Genes, Environment and Alzheimer's Disease, 2016). This process of Aβ plaque formation is associated with multiple neuropathological mechanisms, among which is the hyperphosphorylation of the tau protein and the subsequent formation and accumulation of paired helical filaments inside neurons. The Aβ and tau protein aggregates synergistically trigger a cascade of events that lead to neurite dystrophy, asotrocytosis, disruption of ion homeostasis, oxidative stress and synapse loss. They together cause progressive neuronal death (Winblad et al., Alzheimer's Research and Therapy, 2014 :6: 1-12, Davtyan el al., Alzheimer's Research & Therapy, 2019 :11: 107)(Roda et al., Neural Regeneration Research, 2022 :17: 1666-74).

The US Food and Drug Administration (FDA) has approved very few medications for the treatment of AD. These include donepezil, rivastigmine, galantamine, which are acetylcholinesterase inhibitors, memantine, which is a partial antagonist of the N-methyl-D-aspartate receptor, and the combination of donezepil with memantine. All of these drugs have limited effectiveness and do not reverse disease progression or improve cognitive dysfunction. They only delay the deterioration of symptoms for a limited period (Vassilakopoulou et al., Vaccines, 2021:9: 1-27). Recently the FDA approved two disease-modifying drugs, Aducanumab and Lecanemab. Both are monoclonal antibodies that target aggregated Aβ species. Both are being controversial due to the high incidence of amyloid-related imaging alterations, ARIAs in patients or insufficient evidence of efficacy.

In this context, the development of new disease-modifying therapies for AD remains an urgent need. Most modifying drugs currently in development have Aβ peptides or the Tau protein as their primary targets (Cummings et al., Alzheimer's and Dementia: Translational Research and Clinical Interventions, 2021:7: 1-24). Immunotherapy is one of the most extensively studied strategies. Its mechanism of action is to promote the clearance of malformed proteins from the brain, mediated by antibodies (Yiannopoulou & Papageorgiou, Journal of Central Nervous System Disease, 2020:12: 1-12).

Passive immunotherapy is one of the most active strategies (Cummings et al., Alzheimer's and Dementia: Translational Research and Clinical Interventions, 2021:7: 1-24). However, it has several drawbacks such as the high incidence of adverse reactions, ARIAs, it requires frequent administrations, high doses and high production costs. Finally, there are still no conclusive reports on its effectiveness from a clinical point of view.

Active immunotherapy, on the other hand, is one of the most promising strategies and the best positioned for preventive intervention (Cacabelos, Expert Opinion on Drug Discovery, 2019:15: 1-6). It has advantages over passive immunotherapy because the production of specific antibodies is typical of the immunized patient, and is therefore polyclonal, which makes it potentially multitarget. Furthermore, a sustained antibody response can be induced with a reduced number of immunizations. Recently, a meta-analysis on the efficacy and safety of immunotherapy in clinical trials showed that ARIAs are significantly less frequent in vaccinated patients than in patients treated with monoclonal antibodies (Foroutan et al., Clinical and Investigative Medicine, 2019 :42: E53-65). However, because the evolution of AD is closely associated with the aging process, active immunotherapy has the challenge of developing strategies that stimulate the production of high antibody titers even in the context of the immunosenescence processes that appear in these elderly patients.

The efficacy of active immunization against the Aβ peptide was tested for the first time in the clinic with the AN1792 vaccine, consisting of a synthetic Aβ1-42 peptide (Winblad et al., Alzheimer's Research and Therapy, 2014:6: 1-12). However, phase II clinical trials showed that only 19.7% of patients with mild to moderate AD developed an anti-Aβ antibody response (Vellas et al., Current Alzheimer Research, 2009: 6: 144-51). The appearance of ARIAs forced the end of this trial. These adverse events observed in patients treated with AN-1792 were attributed to the induction of a specific TH-1 (T-helper type 1) cell response generated by T cell epitopes contained in the synthetic peptide Aβ1-42 that are generally located in its central area. However, follow-up of patients who responded to treatment revealed that approximately 4.6 years later, they maintained an anti-AN-1792 antibody response. This response, although with low titers, was persistent despite receiving only 1-3 injections of those initially planned in the phase II study. Furthermore, the progression of cognitive decline was significantly reduced compared to the group of patients treated with placebo, indicating that active immunotherapy may have long-term benefits (Vellas et al., Current Alzheimer Research, 2009:6: 144 -51).

With the aim of avoiding a T cell response, the second generation of vaccines was based on regions of the N-terminal end of Aβ, which mainly contains B cell epitopes. These strategies are based on administering short Aβ peptides, cleaved peptides or mimetic peptides to activate the patient's immune system. They are usually conjugated to a carrier, such as virus-like particles or KLH (keyhole limpet hemocyanin), and administered with an adjuvant to promote stimulation of the immune response (Winblad et al., Alzheimer's Research and Therapy, 2014:6: 1-12). With this new approach, several candidates have reached clinical stages of development, such as CAD106, ACC 001, AD01-03, ACI-24, UB311, Lu AF20513 and ABvac40.

In contrast, active immunotherapy targeting tau protein as a therapeutic strategy for AD is poorly explored. Epitope selection is more complex due to the flexibility of this protein and the abundance of post-translational modifications that both normal and pathological forms undergo (Novak et al., Frontiers in Neuroscience, 2018:12: 1-14). On the other hand, it is not yet known which of its conformations is the most toxic (Meeting & Bu, ALZFORUM, 2020 1-7). Approaches for such selection vary from domains of the tau protein to specific phospho-epitopes. The clinical studies carried out to date are limited, results have only been presented on the candidate AADvac1, and so there is little evidence on its effectiveness in the treatment of AD.

All of these anti-Aβ and anti-tau vaccine candidates have generally been successful in inducing a specific antibody response and acceptable safety profiles, demonstrating the feasibility of active immunotherapy as a promising approach for the treatment of AD. However, there is no conclusive evidence for any of them regarding their effectiveness in stopping the progress of cognitive deterioration; and in some cases, such as AD02 and CAD106, worsening of symptoms compared to placebo has been observed (Francesco et al., Nature Reviews Neurology, 2019 :2: 73-88). All of them have in common that they are focused on a single therapeutic target, but experimental evidence indicates that AD symptoms appear in the presence of both characteristics: the accumulation of Aβ and the loss of tau function (Zampar & Wirths, Peptides in Alzheimer's Disease, 2020 23-50). This, together with the multifactorial and heterogeneous nature of this pathology, suggests that a multitarget strategy would have a greater impact on the progression of the disease.

The solutions of combinations of Aβ and tau epitopes that have been reported so far have not yet advanced to clinical stages, nor has their efficacy been demonstrated in humans. These are described in patents US 201801539733 A1, EP2883955 B1, WO 2021236809 A2 and WO 2022032166. The first of them consists of a DNA vaccine composed of four Aβ1-42 repeats fused to four repeats of the tau379-408 segment. Although no adverse effects were reported in mice, this candidate has the risk of inducing a pro-inflammatory cellular response because it contains the Aβ Th-1 cell epitopes in its structure (Orgogozo et al., Neurology, 2003 :61: 46-54). On the other hand, one of the main concerns regarding DNA vaccines is the possibility that the injected DNA integrates into the cell's chromosomes and has no effect, or, on the contrary, cancer develops in the long term due to the modifications it causes on the DNA of the host cell. Likewise, there is a risk of developing antibodies against the DNA and not against the designed target (Hasson et al., Asian Pacific Journal of Tropical Biomedicine, 2015:5: 344-53). The safety of this proposal has not yet been demonstrated in humans.

The second proposal is a recombinant antigen made up of three copies of the Aβ1-11 fragment and three of the tau2-18 based on the MultiTEP platform with the configuration 3Aβ1-11-MultiTEP-3tau2-18 (EP2883955 B1). However, in animal models anti-tau titers were significantly lower after treatment with 3Aβ1-11-MultiTEP-3tau2-18 compared to treatment with 3tau2-18-MultiTEP or the mixture 3Aβ1-11-MultiTEP and 3tau2-18-MultiTEP. These results, in combination with in silico studies of the three-dimensional structure of the antigens, suggested that the tau epitopes were left with an unfavorable presentation on the surface of the construct proposed in this work (Davtyan et al., Scientific Reports, 2016:6: 28912). In this way, the design of chimeric protein that fuse Aβ and tau epitopes in a single molecule requires strategies that allow their exposure on the surface of the three-dimensional structure so that they can be efficiently recognized by the immune system. Finally, this antigen contains a small segment of tau (tau2-18), which is not specific for the pathological forms of this protein, so the response it prompts probably does not guarantee the effectiveness of the treatment.

The remaining two proposals are very similar in their design (WO 2021236809 A2 and WO 2022032166). Both are based on the fusion of an Aβ1-10 segment copy with a tau244-372 segment copy (a microtubule binding motif). The difference between them consists of adding to this antigen an additional α-synuclein peptide, a key protein in the pathogenesis of Parkinson's disease. Both are based on the classic B epitope region of the Aβ peptide that has so far been used in most immunotherapeutic strategies that have reached clinical stages, which alone has not been shown to be successful in stopping the progression of the disease until now. As for the tau protein, both are based on a region that is characterized by being variable and poorly conserved in the six isoforms of the protein, so that both strategies limit their scope to only a part of the species involved in the pathology.

The four multi-epitope proposals described have in common that none include specific fragments of the pathological forms of the Aβ peptide, such as the C-terminal fragment of Aβ42 and the phosphorylated forms of the tau protein, which are key in the development of the disease. Furthermore, taking into account that pathological tau phosphorylations have a heterogeneous profile and that it depends on the different stages of AD and the brain regions of the patients, it would be more effective if the vaccine candidates were focused on the phosphorylated sites and included a greater number of key epitopes simultaneously or highly conserved immunogenic segments in the six isoforms. All of these elements could lead to treatment with these antigens not having sufficient effect on the elimination of the most toxic species and therefore having limited efficacy.

To date, there is no vaccine candidate whose antigen is composed of a single protein molecule that combines epitopes of both Aβ and tau proteins, which guarantees stimulating a multitarget response with high antibody titers even in the context of patients' immunosenescence, and which manage to prevent or stop the Alzheimer's disease progression.

The present invention solves the problem raised above, by providing an antigen composed of a chimeric protein that comprises the combination of the amino and carboxyl terminal regions of the amyloid beta peptide (Aβ1-42), the amino and carboxyl terminal regions of the tau protein and a T cell epitope. The pharmaceutical composition comprising this chimeric antigen and at least one pharmaceutically acceptable vaccine adjuvant increases the efficacy of AD immunotherapy.

The antigen of the present invention constitutes a unique construction, whose design was based on the selection of segments with immunogenic potential of the Aβ peptide and the tau protein. From the Aβ peptide, the residues included in the Aβ1-12 fragment of the N-terminal end were included because this is an epitope of B cells in humans (Geylis et al., Neurobiol Aging. 2005: 26(5):597-606) and not contains proinflammatory epitopes (Maier M et al., J Neurosci. 2006:3; 26(18):4717-28). Residues comprised in the Aβ28-42 fragment were also included, of which there are no reports to date of its use as an antigen in vaccine candidates for AD. Furthermore, this segment confers specificity to the antigen against the more aggregative Aβ1-42 isoform. The Aβ13-27 segment was excluded from the construct as it contains T cell epitopes responsible for pro-inflammatory adverse effects.

The tau fragments selected to make up the antigen include residues 1-48 and 320-441 because they are potentially successful in stimulating the effective immune response against AD and do not promote an exacerbated cellular inflammatory response (Selenica et al., Journal of Neuroinflammation 2014:11:152). The tau 1-48 segment is a conserved segment in all six isoforms of the tau protein (Wang and Mandelkow, Nature Reviews/Neurosciences, 2016:17:5-21). Furthermore, it contains the regions between amino acids 9-15 and 21-27 at the N-terminus that are highly immunogenic (Selenica et al., Journal of Neuroinflammation 2014:11:152). On the other hand, the fragment comprising tau residues 319-441 is conserved in all six isoforms and contains the highly immunogenic region 427-438 (Selenica et al., Journal of Neuroinflammation 2014:11:152). Additionally, it includes serines 396, 404, and 422 (S396, S404, and S422) that are typically phosphorylated in AD. Phosphoserine 422 (pS422) is specific for pathological tau and is also associated with other neurodegenerative disorders (Troquier et al., Curr Alzheimer Res, 2012:9(4):397-405). Regions 199-441 and 1-368 were discarded because the segment between amino acids 274-315 may favor tau protein aggregation (Wang and Mandelkow, Nature Reviews/Neurosciences, 2016:17:5-21; Šimić et al. Biomolecules, 2016:6(1):6), which is a drawback for the production of vaccine antigens. Furthermore, the protein aggregates would cause the non-exposure of the relevant epitopes to be recognized by the immune system. The tau fragments 1-198, 199-441 and 1-368 were also discarded as they are not conserved for the six isoforms of this protein (Wang and Mandelkow, Nature Reviews/Neurosciences, 2016:17:5-21 ) and all of them have relevance in the development of AD (Buée et al., Brain Research Reviews, 2000:33:95-130).

In one embodiment of this invention, the chimeric antigen comprises the arrangement of tau segments at the N- and C- termini of the chimera such that the antigen retains their positions in the native tau protein. The two segments of Aβ are located in the central region of the chimera. Between the two segments of Aβ is included a T cell epitope.

In one embodiment of this invention, the N- and C-terminal regions of the Aβ peptide comprise 10-15 residues of Aβ1-16 and Aβ28-42 0, respectively; and the N- and C-terminal regions of the tau protein comprise 40-121 residues of the tau1-48 and tau 320-441 fragments; and the T cell epitope is a segment of Tetanus Toxoid (TT). In an embodiment of this invention, the serine residues included in the tau fragment 320-441 corresponding to the sites that are typically phosphorylated in AD are replaced by glutamic acid to simulate post-translational phosphorylation, which guarantees the incorporation of the negative charge that would provide the phosphate group to the molecule, in addition to a larger radical group to favor the adoption of the tertiary structure characteristic of the phosphorylated site. All segments are separated by five amino acids that provide flexible regions and in an arrangement such that, according to in silico studies, all relevant epitopes are exposed on the surface of the polypeptide tertiary structure.

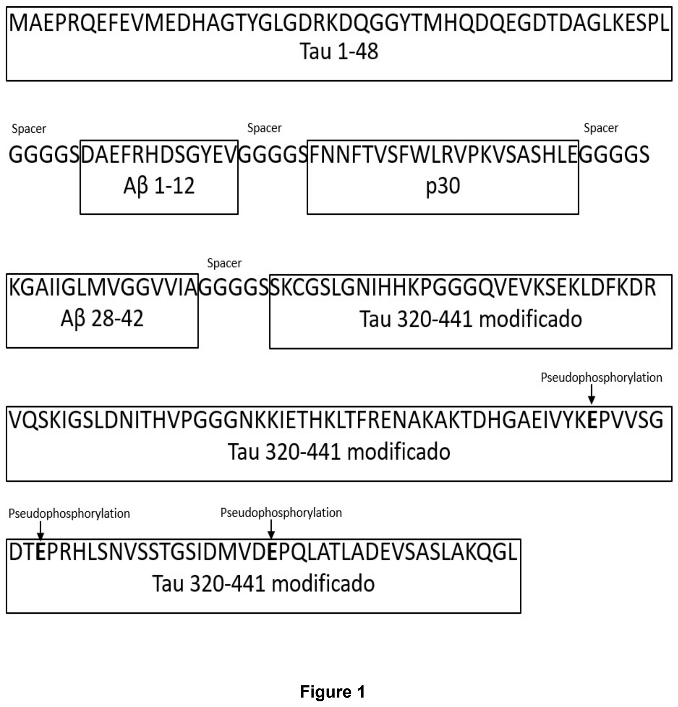

In one embodiment of this invention, the chimeric antigen is characterized by an amino acid sequence that is identified as SEQ ID NO: 1 in the Sequence Listing and is schematized in Figure 1, or an amino acid sequence that has an identity of at least 90% with SEQ ID NO: 1, and hereinafter referred to as AβT. This combination and arrangement of the immunogenic segments selected to form the chimeric antigen of the present invention has not been reported so far.

In one embodiment of the invention, the chimeric antigen also comprises an amino-terminal segment selected from the group of fragments of the LpdA protein of *Neisseria meningitidis,* glutathione-S-transferase, immunoglobulins, ovalbumin, diphtheria toxoid, outer membrane protein complex from meningococcus, protein D from *Haemophilus influenzae,* keyhole limpet hemocyanin. At the carboxyl terminus, said chimeric antigen also comprises a sequence that is selected from the group of poly-His, poly-Arg, FLAG.

In one embodiment of the invention, the antigen identified as SEQ ID NO: 1 is fused at the amino terminus to the first 47 amino acids of the LpdA protein of *Neisseria meningitidis, a* spacer region of 13 amino acids. In the carboxyl terminal region it is fused to a poly-His.

The chimeric antigen of interest for this invention was obtained from the insertion of the corresponding synthetic gene into the expression vector through a ligation reaction with T4 DNA ligase and was subsequently confirmed by sequencing. The chimeric antigen obtained by the method described above can be cloned into expression vectors in viruses, bacteria, yeast, phages, plants or mammalian cells, changing the insertion sites as appropriate. Once the genetic construction has been obtained, its sequence must be verified using automatic sequencing methods.

In a particular embodiment, to obtain the chimeric antigen, the pM238 vector for the Escherichia coli bacteria was used, which has, among other elements, a Trip promoter, the 47 amino acid sequence corresponding to the amino-terminal region of the LpdA protein of N. meningitidis. (Patent EP0816506B1), a segment of six histidines and the T4 terminator. This vector also carries the ampicillin resistance gene, as a selection gene. The chimeric fusion protein obtained was named PK-AβT. Once the polypeptide was purified by metal ion chromatography, it was verified by mass spectrometry analysis that it constituted more than 90% of the species identified with a molecular mass of 32.6 kDa, equal to that expected.

In the present invention, the chimeric antigen fused to the LpdA protein fragment of *Neisseria meningitidis* PK-AβT unexpectedly increased the efficacy of immunization without deteriorating the safety profile of the treatment in relation to the administration of these individual fragments mixture. This combination and novel arrangement of the different segments increased its immunogenic properties with respect to the mixture of individual fragments. It gives the chimeric antigen advantages over other variants of immunotherapy for AD, such as: 1) greater effectiveness in raising high titers of antibodies, 2) stimulates a multitarget response without compromising the safety profile of the treatment, 3) overcomes the typical immunosenescence of elderly patients, 4) requires fewer administrations than passive immunotherapy and 5) facilitates vaccine formulation by requiring the production of a single protein molecule instead of the mixture of individual fragments. These elements provide the chimeric antigen with high potential for the treatment of neurodegenerative diseases that are characterized by the aggregation of the Aβ peptide and the tau protein. The present invention describes specific active immunotherapy characterized by the administration of a recombinant chimeric antigen composed of segments of the Aβ peptide, the modified tau protein and the tetanus toxoid, which is combined with a pharmaceutically acceptable vaccine adjuvant.

In one embodiment of the invention, in the pharmaceutical composition the vaccine adjuvant is selected from the group consisting of oil adjuvants, mineral salts, proteoliposomes and proteoliposomes conjugated to gangliosides. In order to promote the development of the immune response, the chimeric antigen of the present invention can be combined with immunopotentiators and/or adjuvants.

The immunogen can be administered in vehicles accepted for pharmaceutical use that are not toxic or have therapeutic effects, and that are known to those skilled in this branch of the art. The examples presented in this invention do not restrict in any way the use of a particular buffer or combinations of buffers, and even the use of excipients that contribute to increasing their stability.

In another aspect, the invention provides the use of the chimeric antigen comprising the combination of segments of the Aβ peptide, the modified tau protein and a T cell epitope, for the manufacture of a medication for the prevention and treatment of Alzheimer's disease. This is because the combination and arrangement of these segments allows obtaining an antigen with a stable conformation that presents all the epitopes of interest on its surface. This facilitates recognition by the immune system and stimulates a multitarget-specific antibody response against the toxic Aβ peptide and tau protein species. The chimeric PK-AβT fusion protein, administered with aluminum salt adjuvant, gives rise to a vaccine that is capable of breaking the tolerance of the immune system, and inducing a specific humoral and cellular immune response to the Aβ peptide and the tau protein simultaneously. This immune response is superior, qualitatively and quantitatively, if compared to the immunological response generated when a mixture of its peptides is used in the vaccine formulation. This increase in the immunogenicity of the chimeric PK-AβT antigen translates into an increase in the neuroprotective activity of the immune response effectors that is induced, greater than that generated by the other strategies evaluated.

The neuroprotective effects demonstrated in the invention may be based on different mechanisms, without ruling out their possible combinations. The polyclonal antibody response generated by PK-AβT vaccination is capable of clearing the toxic species of both Aβ peptide and tau protein from the brain in a synergistic manner. Clearance is favored because antibodies can block and inhibit the aggregation process during the formation of Aβ plaques and neurofibrillary tangles through target neutralization. Furthermore, they can dissolve aggregates and opsonize them by activating microglia and macrophages. On the other hand, antibodies generated by vaccination can favor the transport of toxic species, through the blood-brain barrier, into the peripheral bloodstream. As a consequence of all this, the neurotoxicity induced by Aβ and tau aggregates decreases. The polyclonal nature of the antibody response, which is induced because of immunization, is an important factor in the effectiveness of the biological events described above.

Immunization with PK-AβT induced a highly polarized response to the Th-2 type, through the majority production of IgG1 isotype antibodies. IgG1 production is an indirect measure of the relative contribution of Th-2 type cytosines, while IgG2a isotypes reflect the contribution to a Th-1 immune response. Furthermore, this Th-2 type cellular response was corroborated by the increase in the production of non-inflammatory cytokines such as IL-4 in the splenocyte cultures of the immunized mice.

All these properties of the immune response generated after immunization with the PK-AβT protein become more relevant when compared with the active immunotherapy strategy with the Aβ and tau fragments separately. An important element is that a higher immune response is achieved with the same amount of antigen. This feature implies that the immunotherapy strategy that involves PK-AβT has advantages in humans, from a productive and safety point of view.

The invention also provides a method for the prevention and treatment of AD. This comprises the administration of a therapeutically effective amount of a pharmaceutical composition comprising a) the chimeric antigen comprising segments of the Aβ peptide, the modified tau protein and the tetanus toxoid, and b) at least one pharmaceutically acceptable vaccine adjuvant. This method, which involves immunization with the aforementioned chimeric antigen, more effectively prevents or decreases the aggregates of the Aβ peptide and the tau protein, which offers advantages to stop the progression of cognitive decline in patients.

In one embodiment of the invention, the method involving administration of the composition comprising the chimeric antigen is combined with standard AD therapy simultaneously or sequentially. This treatment method can be used as first or second line therapy, in association or not with other chemical or biological agents.

In one embodiment of the invention, the administration of the vaccine comprising the chimeric antigen is combined with the bolus injection, or with the controlled release, of monoclonal antibodies or their fragments specific for antigens relevant to the aggregation of the Aβ peptide and the protein tau. Vaccine preparations comprising the chimeric antigen are administered to a human in a therapeutically effective amount. The mixture of antigen and adjuvant, also known as immunogen, can be administered by various routes, including subcutaneous, intramuscular, mucosal, peritoneal, intralymphatic, topical or inhalation. In a preferred embodiment, the administration route of the immunogen comprising the chimeric PK-AβT antigen is subcutaneous. The examples presented in this invention do not restrict the use of the antigen to an adjuvant or by a particular route of administration.

In an embodiment of the invention said treatment or prevention method will be repeated at least once with intervals of between 15 and 28 days. The doses of antigen to be used can be established according to different parameters. These doses depend on the administration route, the disease stage and the treatment period. A change in the dose administration interval, or a different administration route than those described in the examples that follow, do not deviate from the essence of the present invention, being possible to achieve an optimization of the immunization schedules in order to obtain a better specific immune response to PK-AβT.

The following examples and figures, which should not be interpreted in any way as limiting the present invention, illustrate the multitarget action of immunotherapy with the proposed chimeric antigen on the main neuropathological mechanisms of AD.

### Description of the content of the figures

**Figure 1****.** Polypeptide that makes up the chimeric AβT antigen.

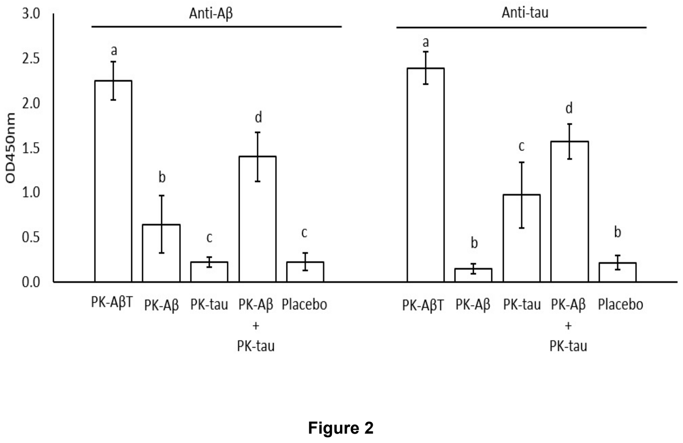

**Figure 2****.** Response of anti-Aβ and anti-tau antibodies generated after immunization of mice with the PK-AβT polypeptide in combination with the aluminum hydroxide adjuvant, measured by ELISA. Antibody levels are expressed as absorbance units at 450 nm for the 1:10,000 dilution of the sera evaluated. Different letters represent statistical significance according to the Tukey test (p<0.05).

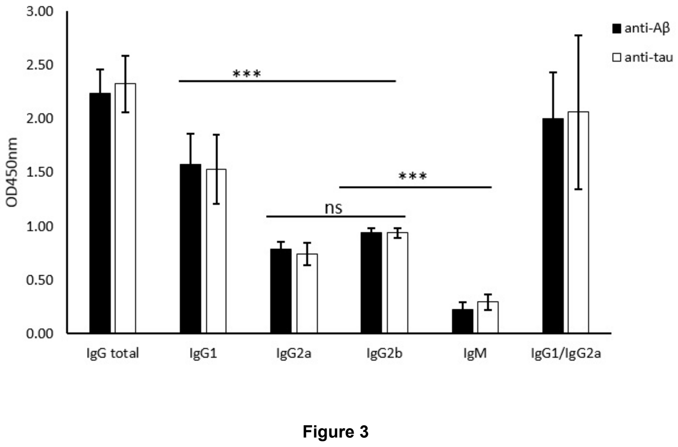

**Figure 3****.** Subclasses of anti-Aβ and anti-tau antibodies generated by vaccination with PK-AβT in combination with the aluminum hydroxide adjuvant, determined by ELISA. Antibody levels are expressed as absorbance units at 450 nm for the 1:10,000 dilution of the sera evaluated. Asterisks represent statistical significance according to Tukey's test (***p<0.001).

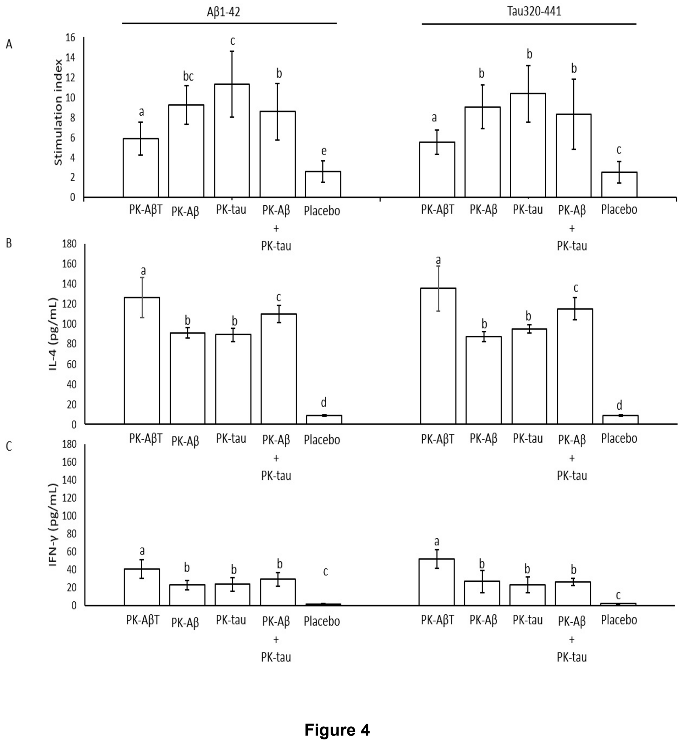

**Figure 4****.** Cellular response induced by vaccination of mice with the chimeric PK-AβT antigen, its individual fragments or a mixture of them in combination with the aluminum hydroxide adjuvant. T cell proliferation (A) and levels of cytokines II-4 (B) and INF-γ (C), measured in the splenocyte cultures of individual mice after their re-stimulation in vitro with the peptides Aβ42, tau320- 441 or p30. Different letters represent statistical significance according to the Tukey test (p<0.05).

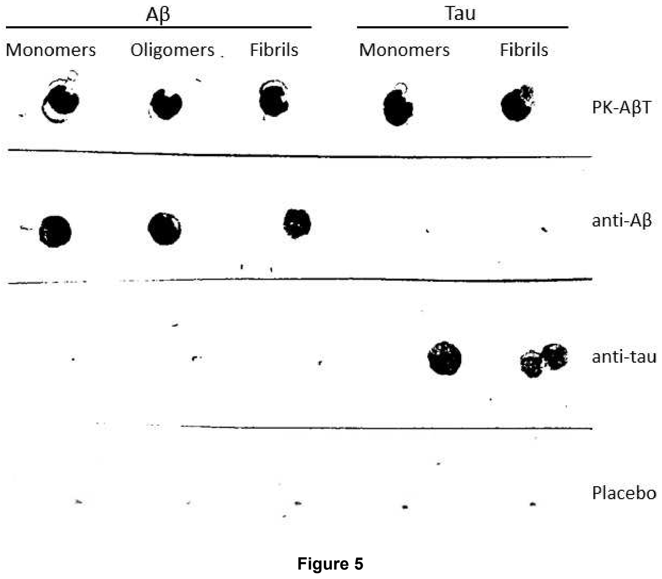

**Figure 5****.** Dot blot showing the reactivity of sera from mice immunized with the PK-AβT polypeptide towards all monomeric and aggregated forms of the Aβ peptide and the human tau protein.

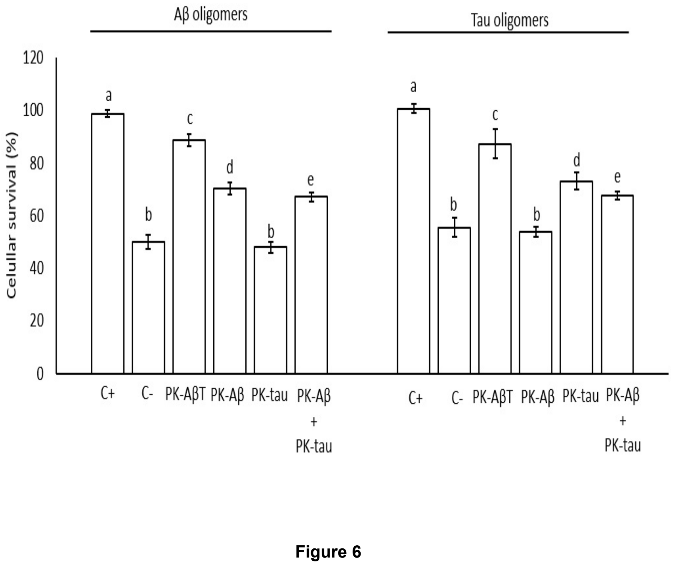

**Figure 6****:** Inhibition of cytotoxicity induced by Aβ or tau oligomers in SH-SY5Y human neuroblastoma cells mediated by antibodies from the serum of mice immunized with the PK-AβT polypeptide or the mixture of its fragments. Different letters represent statistical significance according to the Tukey test (p<0.05).

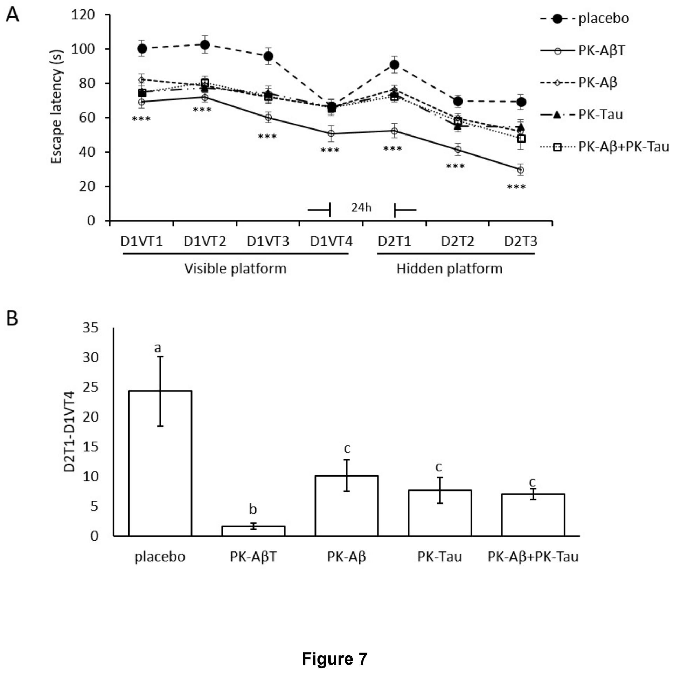

**Figure 7****:** Effect of vaccination with the PK-AβT polypeptide on cognitive deficit in Alzheimer's Disease 3xTg transgenic mice. A: Performance in the Morris Water Maze in the two-day protocol of the different experimental groups. B: Long-term spatial memory, measured as the escape latency of the last trial with the visible platform (D1T4) minus the escape latency of the first trial with the visible platform (D2T1). Different letters represent statistical significance according to the Tukey test; ***p < 0.001.

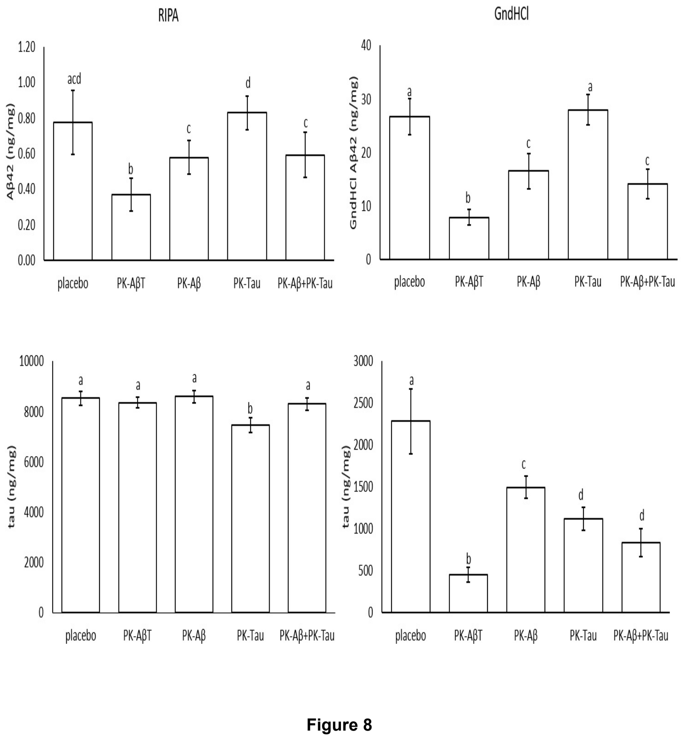

**Figure 8****:** Effect of vaccination with the PK-AβT polypeptide on the deposition of Aβ and tau in the brain of 3xTg transgenic Alzheimer's disease mice, measured as the amount of Aβ42 and tau in the brain fractions of soluble proteins (RIPA) and insoluble (GnHCl). Different letters represent statistical significance according to the Tukey test (p<0.05).

Below are examples of the multitarget chimeric antigen embodiments, which should not be interpreted in any way as limiting the present invention.

### Example 1. Cloning and expression of the chimeric fusion protein

The gene corresponding to the chimeric antigen AβT was synthesized through the services of the Genewiz company (USA). In order to have representative polypeptides of the Aβ peptide and the tau protein separately, two additional genes called Aβ and tau respectively were synthesized. The synthesis of the three genes was carried out taking into account: 1) the optimization of codons for the expression of proteins in *E. coli* systems, and 2) the inclusion of the cutting sites of the *Nhe I* enzymes in the 5' position and *BamH I* in the 3' position of the sequence for assembly in the pM238 vector. The sequence of the three synthetic genes AβT, Aβ and tau was 100% verified by complete sequencing. The nucleotide sequence of the synthetic genes AβT, Aβ and tau are identified in the Sequence Listing as SEQ ID NO: 3-5, respectively. The assembly of the synthetic genes that encode the chimeric antigen AβT and the Aβ and tau fragments separately, in the pM238 vector, was carried out by double digestion with the enzymes *Nhe I* and *BamH I* of both the vector and each gene, the purification of the restriction fragments from the agarose gels with the Monarch DNA Gel Extraction Kit (New England Biolabs) and the subsequent ligation reaction with the enzyme T4 DNA ligase. With the inclusion of the synthetic gene in the pM238 vector, each of the AβT, Aβ and tau antigens was fused to 47 amino acids from the amino terminus of the *N. meningitidis* LpdA protein (Patent EP0816506), connected by 13 amino acids that function as a bridge. Towards the carboxyl terminus of each fusion protein, a region of six histidines was located to facilitate its purification. The plasmids resulting from the cloning of the three synthetic genes were named pPK-AβT, pPK-Aβ and pPK-tau, respectively. The nucleotide sequence of these plasmids was 100% verified. The nucleotide sequence of the coding region for each of the three chimeric fusion proteins is identified in the Sequence Listing as SEQ ID NO: 6-8. The fusion polypeptides were named PK-AβT, PK-Aβ and PK-tau and their amino acid sequences are identified in the Sequence List as SEQ ID NO: 2, 9 and 10, respectively.

For the expression of the three chimeric proteins PK-AβT, PK-Aβ and PK-tau, *E. coli* bacteria of the BL21 strain transformed with the plasmids pPK-AβT, pPK-Aβ or pPK-tau were grown for 3 h at 37 °C in a 5 L fermenter. Luria Bertani medium was used, supplemented with 100 µg/mL ampicillin (pH 7.0-7.5). To induce the expression of the three chimeric proteins, 10 to 100 µg of 3β-indoleacrylic acid were added per mL of medium, and each of the cultures was allowed to grow at 37 °C for 16 hours.

### Example 2. Obtaining, purification and characterization of the chimeric fusion protein

The bacterial biomass obtained by fermentation (as described in Example 1) was subjected to chemical breakdown using phosphate buffer (100 mM NaH₂PO₄, 10 mM Tris-Cl, 8M urea, pH 8). Each of the PK-AβT, PK-Aβ or PK-tau proteins, contained in the soluble fraction, was purified by metal chelate affinity chromatography under denaturing conditions, using a Ni-NTA matrix (Qiagen), and it was then subjected to gel filtration.

To characterize the chimeric fusion proteins PK-AβT, PK-Aβ and PK-tau, western blot techniques were used for the immunodetection of the fragments of Aβ, tau and the LpdA protein fragment of *N. meningitidis;* and ESI-MS mass spectrometry a QTOF-Ultima orthogonal hybrid configuration mass spectrometer for verification of its molecular mass. The presence of the Aβ, tau and the *N. meningitidis* LpdA protein fragments in the three chimeric fusion proteins was verified because they were recognized by a 1:2500 dilution of the anti-Aβ antibody A3981 (Sigma), a dilution of 1 :5000 of the anti-tau MAB 2239 antibody (Sigma) or a 1:4000 dilution of the anti-P64K mab-Ntp64k-HRP antibody (CBSS), respectively. The ESI-MS/MS mass spectra allowed us to verify that the proteins are intact, as designed, and their molecular masses coincide with those expected.

### Example 3. Immunogenicity of the chimeric fusion antigen in mice with Alzheimer's disease

To evaluate the immunogenicity of the chimeric PK-AβT fusion protein, three-month-old male 3xTg-AD transgenic mice were immunized subcutaneously. Furthermore, in this experiment, the chimeric auxiliary proteins PK-Aβ and PK-tau injected separately, as well as the mixture of them (PK-Aβ + PK-tau), were used as controls. In all cases, each mouse received 50 µg of chimeric antigen per dose in aluminum hydroxide as an adjuvant. Other mice were immunized with the mixture of excipient (40 mM Tris pH 7.4) and adjuvant as a negative control. All mice received an injection every two weeks for eight weeks. In total, each mouse received five injections and blood collection was performed one week after the last immunization.

To detect the response of specific anti-Aβ and anti-tau antibodies, indirect ELISA tests were performed. Soluble proteins 2.5 µM Aβ1-42 (Sigma) or 10 µg/ml human tau (Abcam) were immobilized in a 96-well plate at pH 9.7, 4°C overnight. Mouse sera were added to the plate at a 1:10,000 dilution. Detection was performed with a sheep antibody specific to mouse IgG (Sigma) conjugated. To characterize the subclass profile of anti-Aβ and anti-tau antibodies generated by vaccination in mice, anti-IgG1, IgG2ab, IgG2b and IgM conjugated antibodies (Abcam) were used.

The means and variances of the antibody titers were compared between the experimental groups using an ANOVA (p<0.05) with the Statistica 8.0 program tools (Stat Soft.).

Figure 2 shows the results of the IgG antibodies specific to the Aβ peptide and the human tau protein levels evaluation, expressed in absorbance units. The administration of the chimeric PK-AβT antigen in aluminum hydroxide as an adjuvant was able to generate a significantly greater antibody response than that generated by the Aβ and tau fragments separately or the mixture of them with this same adjuvant (p<0.001 ). This result indicates that the chimeric PK-AβT antigen has greater immunogenicity, which can be explained because the chimera presents a primary structure with marked differences in relation to the native structures of the Aβ peptide and the tau protein, which makes it easier to be recognized as foreign by the immune system and manage to break immunological tolerance. Furthermore, PK-AβT generated a multitarget response against the two main structures that are poorly formed in AD.

The majority subclass of antibodies generated by vaccination with the chimeric PK-AβT antigen was IgG1 and the relationship between the IgG1 and IgG2a subclass was always greater than 1 (Figure 3, ANOVA, p<0.001). The production of IgG1 type antibodies is an indirect measure of the relative contribution of Th-2 type cytokines, while IgG2a reflects the contribution of the Th-1 type response. This result indicates that the chimeric antigen PK-AβT induces a highly polarized Th-2 type response. Thus, immunization generated a strong antibody response without contributing to the neuroinflammation characteristic of AD.

### Example 4. Cellular response generated after immunization with the chimeric fusion antigen

To determine whether vaccination with the chimeric PK-AβT antigen induces T cell activation and proliferation, which are key steps in the development of neuroinflammation, we tested mice immunized with both the chimeric PK-AβT antigen, its individual PK- Aβ or PK-tau or a mixture of them (PK-Aβ + PK-tau), the spleen was removed two weeks after the last immunization. Splenocytes were resuspended in RPMI 1640 at 2x106 cells/mL and 100 µL was added to each well of a flat-bottom 96-well plate. Splenocytes were restimulated in vitro with the addition of 100µL of the Aβ42 or tau320-441 peptide at 20 µg/mL dissolved in RPMI 1640 and mixed. After incubation at 37°C for 72 h, 5% CO2, the supernatants were collected and the proliferative response was measured on the plates with the CellTiter 96 Aqueous One Solution Cell Proliferation Assay reagent set (Promega) according to the manufacturer's instructions. The stimulation index was calculated by the ratio of the optical density values (OD490nm) of the wells that contained the cells stimulated by any of the peptides and those of the cells that only contained medium. IL-4 and INF-γ levels were determined in culture supernatants by ELISA using the Mouse IL-4 Quantikine ELISA Kit and Mouse IFN-gamma Quantikine ELISA Kit reagent sets (R&D Systems) according to the manufacturer's instructions. The values obtained for each of the experimental groups were compared using an ANOVA.

The proliferation of T cells in splenocyte cultures from mice treated with the chimeric antigen PK-AβT, its individual fragments PK-Aβ or PK-tau or a mixture of them (PK-Aβ + PK-tau) was moderate in all the treatments tested (Figure 4). However, surprisingly, treatment with the PK-AβT polypeptide showed significantly less T cell proliferation than the rest of the treatments (Figure 4A). The T epitopes in the formulations with the PK-Aβ and PK-tau antigens separately probably have a greater exposure than with respect to the more complex tertiary structure of the PK-AβT antigen, which protects the chimera from generating a pro-inflammatory response.

On the other hand, the higher levels of IL-4 than INF-γ in the supernatants of splenocyte cultures from mice treated with PK-AβT (Figure 4B and C) correspond with a stronger antibody response polarized to the type Th-2 generated by the chimeric antigen compared to that stimulated with its individual fragments or the mixture of them.

### Example 5. Evaluation of the ability of antibodies resulting from vaccination with the chimeric fusion antigen to recognize the toxic Aβ and tau species

The ability of sera from mice immunized with the PK-AβT antigen to recognize the different structures of Aβ and tau was determined by a Dot Blot assay. Polymerization of both the synthetic peptide Aβ1-42 (Sigma) and the recombinant human tau protein (441aa isoform, Abcam) was first induced *in vitro* according to the procedures previously described by (Stine et al., Methods in Molecular Biology, 2011: 670: 13-32) and (Combs et al., Neurobiology of Disease, 2016:94: 18-31), respectively. Briefly, Aβ1-42 monomers were obtained after dissolving the peptide in cold Hexafluoro-2-propanol (HFIP). Then, HIFP was removed by evaporation and Aβ1-42 was dissolved in anhydrous dimethyl sulfoxide (DMSO). Cold water was added to a 5mM aliquot of Aβ to a final concentration of 100µM immediately before use. Aβ oligomers were prepared from the fresh solution of 5mM Aβ42 in DMSO to which cold F12 culture medium was added to a final concentration of 100µM and incubated for 24h at 4°C. Finally, the preparation of Aβ fibrils also began from a fresh solution of 5mM Aβ42 in DMSO to which 10mM HCl was added to a final concentration of 100µM and incubated at 37°C for 24h. On the other hand, the aggregated tau species were obtained after adding arachidonic acid in ethanol to the recombinant tau protein in polymerization buffer (5mM DTT, 100mM NaCl, 100µM EDTA, and 10mM HEPES at pH 7.64), up to a final concentration of 75 µM and allowed to polymerize overnight.

To perform Dot Blot, drops of the solutions containing monomers, oligomers or fibrils of the synthetic peptide Aβ1-42, or monomers or fibrils of the recombinant human tau protein (2µg/drop), were placed directly on a nitrocellulose membrane. The membranes were incubated in 3% skim milk for 1 h at room temperature, followed by washing with PBST. Then, the membranes were incubated separately for 2 h with the pool of sera from mice immunized with the chimeric PK-AβT antigen. Anti-Aβ 6E10 (BioLegend) or anti-tau (DAKO) antibodies were used as positive controls. The membranes were washed with PBST and incubated for 1 h at room temperature with secondary antibodies anti-mouse IgG (Abcam) or anti-rabbit IgG (Abcam) conjugated to peroxidase at a 1:1000 dilution. The reactivity was visualized with the substrate 3,3'-diaminobenzidine (SIGMA).

Immune serum from mice vaccinated with the chimeric PK-AβT antigen effectively recognized monomeric and aggregated species of both Aβ and tau (Figure 5). In this way, the first function of the antibodies generated as a result of treatment with the chimeric PK-AβT antigen was demonstrated, which is their binding to a wide range of Aβ and tau therapeutic targets simultaneously.

Unlike familial AD in which the cause is genetic, sporadic AD is triggered by a deficiency in the clearance of Aβ from the brain. This peptide accumulates through an aggregation pathway involving different species: monomers, oligomers, fibrils and Aβ plaques. Of these, the most toxic species are the oligomers, which cause calcium dysfunction, mitochondrial failure, inflammatory response, oxidative stress, transcriptional impairment, synapse loss and finally neuronal death. Furthermore, Aβ oligomers induce hyperphosphorylation of tau whereby it aggregates forming oligomers that evolve from paired helical filaments to neurofibrillary tangles. Tau oligomers are equally toxic with strong effects on synapses, and in turn induce the formation of Aβ oligomers. In addition to this positive feedback between Aβ and tau aggregation, both proteins act cooperatively to lead to neuronal death (Roda et al., Neural Regeneration Research, and 2022:17: 1666-74). Therefore, the antibodies generated by vaccination with the chimeric antigen PK-AβT have the potential to act both at a systemic level and at a central level to favor the clearance mechanism of the momomeric and oligomeric species of Aβ and tau from the brain and thus avoid their accumulation and subsequent aggregation. As the different aggregation states of Aβ peptides and tau protein coexist in Alzheimer's brains (Roda et al., Neural Regeneration Research, 2022:17: 1666-74), eliminating all these structures simultaneously would have greater neuroprotective effects *in vivo.*

### Example 6. Evaluation of the antibodies, resulting from vaccination with the chimeric fusion antigen, ability to inhibit cytotoxicity induced by Aβ and tau

The role of antibodies generated by vaccination with the chimeric PK-AβT antigen in inhibiting Aβ and tau-induced toxicity in cell cultures was evaluated in vitro by means of the cell viability assay with MTT (3-(4,5-dimethylthiazole). -2-yl)-2,5-diphenyl-2H-tetrazolium bromide). The toxicity models induced by Aβ oligomers (Davtyan et al., Journal of Translational Medicine, 2011:9: 127) and tau oligomers (Sun et al. Journal of Neuroinflammation, 2021:18:131) were used in the human neuroblastoma cell line SH-SY5Y (ATCC, VA). Cells were inoculated in each well of a 96-well plate at 1x104 cells/100µL of medium (45% DMEM, 45% Ham's Modified F12 Medium, 10% FBS and 2mM L-glutamine). The plates were incubated at 37°C for 24h in 5% CO₂. Antibodies from the immune serum of mice vaccinated with the chimeric antigen PK-AβT, PK-Aβ, PK-tau, the mixture of PK-Aβ + PK-tau were purified by affinity chromatography according to the previously described protocol (Mamikonyan et al ., Journal of Biological Chemistry, 2007 :282: 22376-86). For the induced toxicity assay, Aβ or tau oligomers, prepared as described in Example 5, were incubated for 1 h at room temperature with or without the purified antibodies, with occasional shaking. After incubation, the purified oligomer/antibody mixtures were diluted in culture medium until the oligomers and antibodies were at a final concentration of 2µM and 0.2µM, respectively. 100µL of these dilutions were added to the SH-SY5Y cells and incubated for 72h. In parallel, untreated cells were incubated as a control. Viability was then measured in both toxicity models with the MTT Assay Kit (Abcam) according to the manufacturer's instructions. Cell viability was calculated by dividing the absorbance of each well with treated cells by the absorbance of the wells of untreated cells and was expressed as a percentage.

Figure 6 shows that the incubation of serum antibodies from vaccinated mice with the chimeric PK-AβT antigen or the mixture of its peptides (PK-Aβ + PK-tau) significantly protected the cells against toxic stimuli in both, the *in vitro* model of toxicity induced by Aβ and tau oligomers. These results showed that the best cell viability was obtained with the antibodies resulting from vaccination with the chimeric PK-AβT antigen. In this way, the therapeutic strength of vaccination with the chimeric antigen PK-AβT was verified due to the greater protective effects of the antibodies stimulated by the treatment.

### Example 7. Efficacy of the administration of the PK-AβT polypeptide in the treatment of Alzheimer's disease in mice

To evaluate the effect of vaccination with the PK-AβT polypeptide on the cognitive functions of immunized mice, the Morris water maze test was performed (Morris, Journal of Neuroscience Methods, 1984:11(1):47-6). This behavioral model has demonstrated validity as a measure of hippocampus-dependent spatial navigation and reference memory. For it, a two-day protocol is reported, which is based on an initial series of training tests with the visible platform, followed by a memory test 24 h later, but with the platform hidden (Gulinello et al., Behav Brain Res, 2009:196:220-227). Thus, animals that develop a correct spatial strategy move quickly towards the hidden platform area, indicating that they maintain intact long-term memory capacity. In contrast, animals that take a long time to locate the hidden platform have failures in long-term spatial memory, with longer escape latencies.

All mice were transported to the behavioral testing room in their home cages at least 1 day before testing, and experiments were performed between 8:00 a.m. and 12:30 p.m. A pool 120cm in diameter and 51cm deep was used with a water temperature of 24 ± 2°C, in which high-contrast visual signals were placed on the wall in each quadrant and external signals in the room. The training phase comprised four trials with the visible platform (V1-V4) and the last trial was considered their baseline post-habituation escape latency. This was named D1VT4 (day 1, visible test platform 4). The animals training was staggered in time so that each mouse had the same interval between trials, that is, 30 ± 10 minutes between each test and was started on each occasion from different randomly selected positions in the maze.

In order to make the platform visible, it was identified with a high contrast object with respect to the bottom of the tank. Animals that were unable to escape within 1 minute were manually guided onto the platform and allowed to remain on it for 5-10 seconds before being removed. Subsequently, they were dried and placed in their respective cages illuminated with incandescent light to avoid hypothermia. 24 hours after the last visible platform test (D2), animals were tested in a series of three trials with the hidden platform (T1-T3). The tests were carried out every 30 minutes and each test lasted a maximum of 1 minute. For all tests the platform remained in the same place. The difference in escape latencies was calculated: that of the last trial of training with the visible platform and that of the first trial with the hidden platform (24 h later).

Escape latencies in the training phase with the visible or hidden platform were analyzed using an ANOVA. For the study of long-term memory, the last escape latency value of the training phase with the visible platform (D1VT4) and the first value of the test with the hidden platform performed 24 h later (D2T1) were used. Mice with intact long-term memory capacity must quickly reach the hidden platform on the first trial. A scoring system was generated with the D2T1-D1VT4 difference, which in a successful subject should be close to 0. Higher values indicate a longer escape latency after 24h, which implies a deficit in the spatial performance of the animals.. This comparison was performed using an analysis of variance, followed by Tukey's post-hoc test with a significance level α=0.05.

After the behavioral tests, the animals were sacrificed and the brain was removed immediately to analyze the deposition of Aβ and tau. The extraction of brain proteins was carried out by serial fractionation of the brain homogenate in (1) TBS (50 mM Tris-Cl, pH 7.5, 150 mM NaCl), (2) RIPA (150 mM NaCl, 1% Nonidet P-40, 0.5% DOC, 0.1% SDS, 50 mM Tris pH 7.4) and (3) GndHCl (5M guanidium hydrochloride, 50mM HEPES (pH 7.3), 5mM EDTA) as previously described (Wu et al., Neuropharmacology, 2018 :131: 351-63). Briefly, frozen tissue was homogenized in nine volumes of TBS (m/v) containing protease inhibitors (Roche). The samples were centrifuged for 2h at 16,000g at 4°C and the soluble fraction of TBS was stored in aliquots at -70°C. The pellet was washed in TBS and then resuspended in nine volumes of RIPA (m/v) containing protease and phosphatase inhibitors (Roche). It was mixed gently at 4°C for 30min and then centrifuged for 2h at 16,000g at 4°C. The soluble fraction of RIPA was stored at -70°C in aliquots. The pellet was washed with RIPA and resuspended in GndHCl at a rate of 150 mg of pellet/mL. Mix gently at room temperature for 2 h with occasional vortexing. The samples were centrifuged for 2h at 16,000g at 4°C and the soluble fraction of GndHCl was neutralized with 20 volumes of 1M Tris-HCl. The deposition of Aβ42 and tau in the soluble fractions of RIPA and GndHCl was measured by ELISA using the Amyloid beta 42 ELISA Kit (Invitrogen) and Tau ELISA Kit (Invitrogen), respectively, according to the manufacturer's recommendations. The concentration of Aβ42 and tau in the brain fractions resulting from the different treatments was compared using an ANOVA with a significance level of 0.05.

Figure 7 (A and B) shows the results of behavioral evaluation after vaccination of 3xTg-AD transgenic mice with the PK-AβT polypeptide. The results indicate that mice receiving placebo have long-term spatial memory deficits, and that treatment with PK-AβT, PK-Aβ, PK-tau or PK-Aβ+PK-tau polypeptides significantly improves cognitive loss. The arithmetic difference in the latency time of the last training test with the visible platform and that of the first test with the hidden platform demonstrate that the group vaccinated with the PK-AβT polypeptide was significantly shorter than the rest of the treatments (Figure 7B). These results indicated that this treatment allows the recovery of spatial learning and memory functions in Alzheimer's type transgenic mice and is more effective than individual peptides or a mixture of them.

The analysis of the brain levels of Aβ42 and tau of the 3xTg-AD mice after treatment is shown in Figure 8. These results show that treatment with the PK-AβT polypeptide significantly decreases the load of Aβ42 (the aggregative form of Aβ) of the brain because its levels are decreased both in the RIPA fractions (soluble proteins) and in the GndHCl fractions (insoluble proteins) when compared with the rest of the treatments tested or the placebo. Surprisingly, the levels of soluble tau were not affected by the treatment, but not those of insoluble tau (Figure 8). Immunotherapy significantly reduced the deposition of insoluble tau in the brain of transgenic mice and the result with the chimeric PK-AβT antigen was better than the rest of the treatments (Figure 8). This result showed that treatment with the polypeptide preferentially eliminated toxic tau species, maintaining normal levels of functional tau protein.

### Example 8. Evaluation of the safety profile after administration of the PK-AβT polypeptide and the aluminum hydroxide adjuvant

Female Balb/c mice (n=5) and female New Zealand rabbits (n=4) were immunized subcutaneously, with 200 µg of the chimeric PK-AβT polypeptide with 0.7 mg of aluminum phosphate adjuvant. The doses were administered every 14 days, for four immunizations. In each experiment, a placebo group was included, which was immunized with the excipient of the vaccine antigen formulation and the adjuvant.

In the experimental block corresponding to mice, three additional passive immunotherapy groups were included. One group was administered a mouse monoclonal antibody specific to the Aβ peptide. Another group was given a mouse monoclonal antibody specific to the tau protein. The third group was administered an unrelated antibody control of the same isotype. These antibodies were previously obtained using traditional hybridoma technology. Administration was carried out in all cases with a dose of 12.5 mg/Kg intraperitoneally, with a weekly frequency, for four doses.

In both studies, weekly body weight assessments were performed, at times other than immunizations. Once the immunizations were completed, the biological samples were extracted, which could be carried out in 100% of the animals included in the experiment, in both animal species.

In the case of the mice, at the end of the immunization schedule, the heart, lung, spleen and ten other organs were removed. A qualitative evaluation was carried out on the organs, both macroscopic and microscopic. For microscopic evaluation and histological diagnosis, the samples were processed by well-known methods. It was considered a treatment-related adverse event when alterations were found in the active or passive immunized groups that were not found in the corresponding placebo group.

In the case of rabbits, two blood draws were performed at different times, and each blood draw was used to obtain plasma (for hematology determinations) and serum (for clinical biochemistry determinations). The extractions were carried out before starting the immunization, and one week after the last immunization. Hematology determinations included the count and/or percentage of various cell types, among other parameters. Blood biochemistry measurements included hemoglobin concentration, alanine aminotransferase enzyme concentration, aspartate aminotransferase enzyme concentration, and creatinine concentration, among others. For each parameter, comparisons were made between the values obtained before and after immunizations, between the treated group and the corresponding placebo group, and whether these values were within the physiological range reported for the species. It was considered a treatment-related adverse event when alterations were found in the groups immunized with the PK-AβT polypeptide that were not found in the corresponding placebo group.

No macroscopic or microscopic alterations were observed in the organs of the mice belonging to the placebo groups or in those immunized with the PK-AβT polypeptide. The hematology and clinical biochemistry results obtained in rabbits indicated the absence of significant changes when the determinations made before and after immunization with the chimeric PK-AβT antigen were compared. No significant differences were found between the immunized group and its corresponding placebo group. In general, all hematology and biochemistry parameters for the immunized and placebo groups were within the physiological range. In both mice and rabbits, there were no significant changes in the body weight of the immunized groups with respect to that found in the corresponding placebo groups, at the different times that were evaluated.

The overall analysis of these results demonstrates that immunization with the chimeric polypeptide PK-AβT, in aluminum phosphate adjuvant, generates a safe immune response. This active immunotherapy strategy offers advantages in relation to the passive immunotherapy strategy with specific monoclonal antibodies, due to the absence of adverse effects.

### SEQUENCE LIST

SEQ ID NO: 1 
MAEPRQEFEVMEDHAGTYGLGDRKDQGGYTMHQDQEGDTDAGLKESPLGGGGSDAEF
RHDSGYEVGGGGSFNNFTVSFWLRVPKVSASHLEGGGGSKGAIIGLMVGGVVIAGGGGS
SKCGSLGNIHHKPGGGQVEVKSEKLDFKDRVQSKIGSLDNITHVPGGGNKKIETHKLTFRE
NAKAKTDHGAEIVYKEPVVSGDTEPRHLSNVSSTGSIDMVDEPQLATLADEVSASLAKQGL
SEQ ID NO: 2 
MVDKRMALVELKVPDIGGHENVDIIAVEVNVGDTIAVDDTLITLDLDHDDDDDKASDLLAMA
EPRQEFEVMEDHAGTYGLGDRKDQGGYTMHQDQEGDTDAGLKESPLGGGGSDAEFRH
DSGYEVGGGGSFNNFTVSFWLRVPKVSASHLEGGGGSKGAIIGLMVGGVVIAGGGGSSK
CGSLGNIHHKPGGGQVEVKSEKLDFKDRVQSKIGSLDNITHVPGGGNKKIETHKLTFRENA
KAKTDHGAEIVYKEPVVSGDTEPRHLSNVSSTGSIDMVDEPQLATLADEVSASLAKQGLG
SRAHHHHHH
SEQ ID NO: 3 
gctagccatggccgaaccgcgccaagaattcgaagtgatggaagatcacgccggcacctatggtctgggcgatcgcaaag
atcaaggtggttataccatgcaccaagatcaagaaggtgataccgatgccggtttaaaagaaagtccgctgggtggtggcggt
agtgatgccgaatttcgccatgacagcggctatgaagttggcggcggcagctttaacaatttcaccgttagcttttggctgcg
cgtgccgaaggttagcgcaagccatctggaaggtggcggtagcaaaggtgccatcattggtctgatggtgggcggtgttg
tgatcgctggtggtggtggtagcagcaaatgcggttctttaggcaacattcatcacaaaccgggtggcggtcaagttgaggtga
aaagcgagaagctggacttcaaggaccgcgttcagagcaaaatcggctctttagacaacattacccatgtgccgggcggcg
gcaacaagaaaattgagacccacaagctgacctttcgcgaaaacgcaaaggccaaaaccgatcacggtgccgagatcgtt
tacaaagagccggtggtgagtggtgataccgaaccgcgtcatttaagcaatgtgagcagcaccggcagcattgatatggttga
tgaaccgcagctggccactttagcagatgaagtgagcgcctctttagccaaacaaggtctgggatcc
SEQ ID NO: 4 
gctagccggtggtggcggtagtgatgccgaatttcgccatgacagcggctatgaagttggcggcggcggcagctttaacaattt
caccgttagcttttggctgcgcgtgccgaaggttagcgcaagccatctggaaggtggcggcggtagcaaaggtgccatcattg
gtctgatggtgggcggtgttgtgatcgctggatcc
SEQ ID NO: 5 
gctagccatggccgaaccgcgccaagaatttgaggtgatggaggaccatgccggtacctatggtttaggtgatcgtaaggatc
aaggtggctataccatgcatcaagatcaagaaggtgataccgatgctggtctgaaagaaagtccgctgggtggcggcggcag
 
ctttaacaactttaccgtgagcttttggctgcgtgtgccgaaagtgagcgccagccatctggaaggtggcggtggcagcagtaa
atgcggctctttaggcaatattcaccataaaccgggcggtggtcaagttgaagtgaagagcgagaaactggacttcaaagacc
gcgtgcagagcaaaatcggctctttagacaacattacccatgtgccgggtggcggcaacaagaaaattgagacccataaact
gaccttccgcgaaaacgccaaagccaaaaccgatcatggcgccgagatcgtgtataaagaaccggtggtgagcggcgata
cagaaccgcgtcatctgagcaatgttagcagcaccggcagcatcgatatggtggatgagcctcagctggccactttagccgac
gaagttagcgcctctttagccaaacaaggtctgggatcc
SEQ ID NO: 6 
atggtagataaaagaatggctttagttgaattgaaagtgcccgacattggcggacacgaaaatgtagatattatcgcggttgaa
gtaaacgtgggcgacactattgctgtggacgataccctgattactttggatctagatcacgatgacgatgacgataaagcttcag
atctgctagccatggccgaaccgcgccaagaattcgaagtgatggaagatcacgccggcacctatggtctgggcgatcgca
aagatcaaggtggttataccatgcaccaagatcaagaaggtgataccgatgccggtttaaaagaaagtccgctgggtggtgg
cggtagtgatgccgaatttcgccatgacagcggctatgaagttggcggcggcagctttaacaatttcaccgttagcttttggc
tgcgcgtgccgaaggttagcgcaagccatctggaaggtggcggcggtagcaaaggtgccatcattggtctgatggtgggcgg
tgttgtgatcgctggtggtggtggtagcagcaaatgcggttctttaggcaacattcatcacaaaccgggtggcggtcaagttgag
gtgaaaagcgagaagctggacttcaaggaccgcgttcagagcaaaatcggctctttagacaacattacccatgtgccgggcg
gcggcaacaagaaaattgagacccacaagctgacctttcgcgaaaacgcaaaggccaaaaccgatcacggtgccgagat
cgtttacaaagagccggtggtgagtggtgataccgaaccgcgtcatttaagcaatgtgagcagcaccggcagcattgatatgg
ttgatgaaccgcagctggccactttagcagatgaagtgagcgcctctttagccaaacaaggtctgggatcccgggcacaccat
caccatcaccat
SEQ ID NO: 7 
atggtagataaaagaatggctttagttgaattgaaagtgcccgacattggcggacacgaaaatgtagatattatcgcggttgaa
gtaaacgtgggcgacactattgctgtggacgataccctgattactttggatctagatcacgatgacgatgacgataaagcttcag
atctgctagccggtggtggcggtagtgatgccgaatttcgccatgacagcggctatgaagttggcggcggcggcagctttaaca
atttcaccgttagcttttggctgcgcgtgccgaaggttagcgcaagccatctggaaggtggcggcggtagcaaaggtgccatca
ttggtctgatggtgggcggtgttgtgatcgctggatcccgggcacaccatcaccatcaccat
SEQ ID NO: 8 
atggtagataaaagaatggctttagttgaattgaaagtgcccgacattggcggacacgaaaatgtagatattatcgcggttgaa
gtaaacgtgggcgacactattgctgtggacgataccctgattactttggatctagatcacgatgacgatgacgataaagcttcag
atctgctagccatggccgaaccgcgccaagaatttgaggtgatggaggaccatgccggtacctatggtttaggtgatcgtaag
gatcaaggtggctataccatgcatcaagatcaagaaggtgataccgatgctggtctgaaagaaagtccgctgggtggcggcg
gcagctttaacaactttaccgtgagcttttggctgcgtgtgccgaaagtgagcgccagccatctggaaggtggcggtggcagca
 
gtaaatgcggctctttaggcaatattcaccataaaccgggcggtggtcaagttgaagtgaagagcgagaaactggacttcaaa
gaccgcgtgcagagcaaaatcggctctttagacaacattacccatgtgccgggtggcggcaacaagaaaattgagacccat
aaactgaccttccgcgaaaacgccaaagccaaaaccgatcatggcgccgagatcgtgtataaagaaccggtggtgagcgg
cgatacagaaccgcgtcatctgagcaatgttagcagcaccggcagcatcgatatggtggatgagcctcagctggccactttag
ccgacgaagttagcgcctctttagccaaacaaggtctgggatcccgggcacaccatcaccatcaccat
SEQ ID NO: 9 
MVDKRMALVELKVPDIGGHENVDIIAVEVNVGDTIAVDDTLITLDLDHDDDDDKASDLLAG
GGGSDAEFRHDSGYEVGGGGSFNNFTVSFWLRVPKVSASHLEGGGGSKGAIIGLMVGG
VVIAGSRAHHHHHH
SEQ ID NO: 10

MVDKRMALVELKVPDIGGHENVDIIAVEVNVGDTIAVDDTLITLDLDHDDDDDKASDLLAMA
EPRQEFEVMEDHAGTYGLGDRKDQGGYTMHQDQEGDTDAGLKESPLGGGGSFNNFTV
SFWLRVPKVSASHLEGGGGSSKCGSLGNIHHKPGGGQVEVKSEKLDFKDRVQSKIGSLD
NITHVPGGGNKKIETHKLTFRENAKAKTDHGAEIVYKEPVVSGDTEPRHLSNVSSTGSIDM
VDEPQLATLADEVSASLAKQGLGSRAHHHHHH

## Claims

1. A chimeric protein for the immunotherapy of Alzheimer's Disease that comprises the combination of epitopes of the N- and C-terminal regions of the amyloid beta peptide (Aβ), the N- and C-terminal regions of the tau protein and a T cell epitope.

2. The chimeric protein of claim 1 wherein the N- and C-terminal regions of the amyloid beta peptide (Aβ) comprise 10-15 residues of the fragments Aβ1-16 and Aβ28-42 and the N- and C-terminal regions of the tau protein comprise 40-121 residues of the tau1-48 and tau 320-441 fragments.

3. The chimeric protein of claim 2 wherein the T cell epitope is the p30 peptide of tetanus toxoid.

4. The chimeric protein of claim 3 **characterized by** an amino acid sequence that is identified as SEQ ID NO: 1 or an amino acid sequence that has at least 90% identity with SEQ ID NO: 1

5. The chimeric protein of claim 4 comprising an amino terminal segment selected from group (i) and a carboxyl terminal segment selected from group (ii):
i) Fragments of the LpdA protein of *Neisseria meningitidis,* glutathione-S-transferase, immunoglobulins, ovalbumin, diphtheria toxoid, protein complex of the outer membrane of menignococcus, protein D of *Haemophilus influenzae,* keyhole limpet hemocyanin,
ii) Poly-His, poly-Arg, FLAG,

6. The chimeric protein of claim 5 wherein the amino terminal segment is **characterized by** the first 47 amino acids of the *Neisseria meningitidis* LpdA protein and the carboxyl terminal segment is a poly-His.

7. A nucleic acid molecule **characterized by** a nucleotide sequence that encodes the chimeric proteins of claims 1-6.

8. A pharmaceutical composition comprising a) the antigen of claim 1-7 and b) at least one pharmaceutically acceptable vaccine adjuvant.

9. The composition of claim 8 wherein the vaccine adjuvant is selected from the group of oil adjuvants, mineral salts, proteoliposomes and proteoliposomes conjugated to gangliosides.

10. The composition of claim 9 wherein the vaccine adjuvant is an aluminum salt selected from the group of aluminum hydroxide, aluminum phosphate, aluminum sulfate

11. The composition of claim 10 wherein the vaccine adjuvant is aluminum hydroxide.

12. Use of the chimeric antigen comprising claims 1-7 for the manufacture of a medicine for the prevention and treatment of Alzheimer's disease.

13. The use of the chimeric antigen comprising claims 1-7 wherein the medicament for the prevention and treatment of Alzheimer's disease is a vaccine for active immunotherapy.

14. A method of treating or preventing Alzheimer's disease comprising administering the pharmaceutical composition of claims 8-10 wherein it is administered at least once.

15. The treatment method of claim 14 comprising repeating the administration at least once at an interval of between 15 and 28 days.
